# EUROPÄISCHE PATENTSCHRIFT

(11) **EP 3 846 729 B1**
(45) Veröffentlichungstag und Bekanntmachung des Hinweises auf die Patenterteilung: **17.12.2025**
(21) Anmeldenummer: 19765680.4
(22) Anmeldetag: 04.09.2019
(51) Int. Cl.: A61B 50/20, A61B 50/30, A61B 90/98

(54) **VERPACKUNG FÜR EIN MEDIZINTECHNISCHES WERKZEUG MIT AUTOMATISCHER WERKZEUGERKENNUNG UND VERFAHREN ZUM VERPACKEN MIT EINER SOLCHEN VERPACKUNG**
PACKAGE FOR A MEDICAL TOOL WITH AUTOMATIC TOOL RECOGNITION, AND PACKING METHOD USING SUCH A PACKAGE
EMBALLAGE POUR UN OUTIL UTILISÉ EN TECHNIQUE MÉDICALE, À IDENTIFICATION AUTOMATIQUE D'OUTIL ET PROCÉDÉ D'EMBALLAGE AVEC UN TEL EMBALLAGE

(30) Priorität: 05.09.2018 DE 102018121682
(43) Veröffentlichungstag der Anmeldung: 14.07.2021
(73) Patentinhaber: Aesculap AG, 78532 Tuttlingen (DE)
(72) Erfinder: LENZENHUBER, Frederick, 78532 Tuttlingen (DE); HÖGERLE, Roland-Alois, 78532 Tuttlingen (DE)
(74) Vertreter: Winter, Brandl - Partnerschaft mbB
(86) Internationale Anmeldenummer: PCT/EP2019/073579
(87) Internationale Veröffentlichungsnummer: WO 2020/049044

(56) Entgegenhaltungen:
- WO-A1-2018/013413
- WO-A1-2018/052966
- CN-A- 108 304 897
- DE-A1- 102007 016 537
- DE-A1- 102013 004 168
- DE-U1- 202007 004 638
- DE-U1- 202011 050 118
- US-A1- 2006 109 105
- US-A1- 2007 215 507
- US-A1- 2009 266 728
- US-A1- 2013 092 564
- US-A1- 2017 224 859

## Beschreibung

Die vorliegende Erfindung betrifft eine Verpackung, insbesondere eine Sterilverpackung, für ein medizintechnisches Werkzeug mit einem Aufnahmeraum für das medizintechnische Werkzeug. Sie betrifft außerdem ein Verfahren zum automatischen Identifizieren eines medizintechnischen Werkzeugs mittels einer Verpackung nach der Erfindung.

Es sind verschiedene Verpackungen und Vorrichtungen für medizintechnische Produkte und insbesondere Werkzeuge bekannt. Verpackungen von medizintechnischen Produkten wie Implantaten und Werkzeugen mit spitzen und/oder scharfen Wirkabschnitten, beispielsweise in Form von Schneid- oder Sägekanten, Fräsköpfen und Bohrern oder Gewindeabschnitten, unterliegen besonderen Anforderungen. Denn neben dem Schutz von Anwendern vor Verletzungen vor und insbesondere nach einer Verwendung des Produkts ist außerdem ein Schutz des verpackten Produkts vor ungewünschten Einflüssen wie Verschmutzung und Beschädigung sicherzustellen.

Eine weitere Anforderung an die Verpackung ist eine geeignete Kenntlichmachung des darin verpackten Produkts, was insbesondere wichtig ist, wenn das verpackte Produkt als solches nicht sichtbar ist (da zum Beispiel von der Verpackung verdeckt) oder das Produkt nicht erkennbar und/oder identifizierbar ist (weil zum Beispiel bestimmte Identitätsmerkmale wie z.B. Material, Abmessung oder ähnliches dem Produkt nicht ohne weiteres angesehen werden können). Eine sichere und eindeutige Identifizierung eines medizintechnischen Produkts, wie zum Beispiel eines Operationsinstruments, eines chirurgischen Werkzeugs oder eines Implantats, ist allerdings im Sinne der Patientensicherheit von größter Wichtigkeit.

Ein weiterer wichtiger Aspekt bei medizintechnischen Werkzeugen kann die Weitergabe und Vermittlung werkzeugspezifischer Informationen sein. Dies können zum Beispiel Informationen darüber sein, ob ein bestimmtes Werkzeug für eine bestimmte Anwendung geeignet ist und/oder wie sein Zustand ist. Zum Beispiel ist es im Falle von medizintechnischen Werkzeugen oft wesentlich, ob und wie oft es schon verwendet wurde, welche Standzeit es hat und wie seine Lagerbestände sind. Derartige Informationen sind mittels üblicher Verpackungen nicht oder nur schwierig zu vermitteln und zu pflegen.

Stand der Technik ist, das verpackte Werkzeug mittels an oder in der Verpackung angebrachter Labels für den Nutzer erkennbar zu kennzeichnen. Dabei ist es von Nachteil, dass der Nutzer ohne gesonderte und sorgfältige Sichtung des Labels und/oder der Verpackung nicht ohne weiteres mit Sicherheit erkennen kann, welches Werkzeug sich in der Verpackung befindet. Außerdem sind bei solchen Labels zusätzliche Informationen, wie zum Beispiel maximale Verwendbarkeit, Abmessungen, Materialen etc., unter Umständen nur schwer und mit unbedingter Sorgfalt anbieterseitig darzustellen und nutzerseitig zu erfassen, begründet beispielsweise durch Verschmutzung oder Abnutzung. Hinzu kommt, dass die Handhabung insbesondere von sterilen Produkten wie chirurgischen Instrumenten und Werkzeugen bei der Entnahme aus der Verpackung Beschränkungen (wie Zeitdruck oder Stress bei einer OP) unterliegen kann, die eine nutzerseitige Erfassung und/oder Dokumentation von produktspezifischen Daten erschweren können. Dies ist insbesondere deswegen kritisch, da eine fehlerhafte Verwendung von medizinischen Werkzeugen infolge einer unzureichenden Erfassung des jeweiligen Werkzeugs und dessen Eigenschaften oder Verwendungscharakteristika schnell zu schwerwiegenden negativen Folgen führen kann, insbesondere für einen Patienten. Schließlich bieten bekannte Verpackungen keine oder nur eine beschränkte Möglichkeit einer dokumentierten Werkzeugerkennung.

Aus dem Stand der Technik sind Verpackungen bekannt, in denen das medizintechnische Werkzeug fest und/oder lagedefiniert gehalten ist. Ein Beispiel für solche Verpackungen ist eine Blisterverpackung mit einer Klemmung im Kupplungsbereich des Werkzeugs, um dieses in einer definierten Lage in der Verpackung zu positionieren und zu halten. Eine solche Verpackung ist zum Beispiel aus der DE10 2013 004 168 A1 offenbart, bei der das Operationswerkzeug unter sterilen Bedingungen in einer Kunststoffverpackung verpackt ist, die Verpackung mindestens zweiteilig ausgebildet ist und aus einer inneren Schutzverpackung für empfindliche Teile des Operationswerkzeugs und einer die Schutzverpackung aufnehmenden, siegelfähigen Blisterverpackung besteht. Außerdem wird ein Verfahren zur Verpackung von sterilen Operationswerkzeugen vorgeschlagen, bei dem das Operationswerkzeug unter sterilen Bedingungen in der Kunststoffverpackung verpackt wird, wobei in einem ersten Schritt zunächst das Operationswerkzeug mindestens teilweise und insbesondere mit seinen empfindlichen Schneidkanten und Arbeitsflächen in der ersten Schutzverpackung verpackt wird und die Schutzverpackung zusammen mit dem dort verpackten Operationswerkzeug in eine zweite Blisterverpackung verbracht wird, dort lagengesichert festgelegt wird und danach mit einer Siegelfolie verschlossen wird. Bei dieser Verpackung besteht der Nachteil, dass zwar die Wirkkanten des Werkzeugs sehr gut geschützt sind, das Werkzeug an sich jedoch durch die Verpackung nicht oder nur schwierig für einen Nutzer identifizierbar ist.

Aufnahmevorrichtungen für medizintechnische Werkzeuge, welche auslesbare Datenträger mit werkzeugspezifischen Daten aufweisen, sind bereits aus der WO 2018/013413 A1, der US 2007/215507 A1, der DE 10 2007 016537 A1, der WO 2018/052966 A1, der US 2006/109105 A1, der US 2017/224859 A1, der DE 20 2007 004638 U1, der US 2013/092564 A1 und der US 2009/266728 A1 bekannt. Die DE 20 2011 050118 U1 offenbart ein chirurgisches Behälterinhaltdetektionssystem. Die CN 108 304 897 A offenbart einen Sterilbehälter.

Vor diesem Hintergrund liegt der vorliegenden Erfindung die Aufgabe zugrunde, die genannten Nachteile des Stands der Technik zu verringern, insbesondere eine Verpackung für ein medizintechnisches Werkzeug bzw. eine zur Anordnung in der Verpackung vorgesehenen Wekzeugauflagerung zur Verfügung zu stellen, mit der eine Erkennung des jeweils verpackten Werkzeugs einfach und sicher möglich ist und das Risiko von fehlerhaften Anwendungen des Werkzeugs minimiert werden kann.

Diese Aufgabe wird nach der vorliegenden Erfindung gelöst durch eine Verpackung nach Anspruch 1, also eine Verpackung, insbesondere eine Sterilverpackung, für ein medizintechnisches Werkzeug, mit: einer als Blisterverpackung ausgebildeten Umverpackung, welche eine Unterschale mit einer einen Aufnahmeraum für das medizintechnische Werkzeug ausbildenden Vertiefung und eine an der Unterschale angeordnete Deckelfolie zum Verschließen des Aufnahmeraums aufweist; einer in dem Aufnahmeraum angeordneten Haltevorrichtung, in der das Werkzeug gehalten ist; und einem auslesbaren Datenträger mit werkzeugspezifischen Daten, der an der Haltevorrichtung angeordnet ist. Die vorstehende Aufgabe wird außerdem gelöst durch ein Verfahren zum automatischen Identifizieren eines medizintechnischen Werkzeugs mittels einer Verpackung nach der Erfindung, insbesondere mittels einer Verpackung nach einem der angehängten Ansprüche, umfassend die folgenden Schritte:
- anbieterseitiges Einbringen des Werkzeugs in die Haltevorrichtung und Einbringen der Haltevorrichtung in den Aufnahmeraum der Verpackung,
- anbieterseitiges Beschreiben des Datenträgers mit werkzeugspezifischen Daten,
- anbieterseitiges Verschließen der Verpackung,
- nutzerseitiges Öffnen der Verpackung,
- nutzerseitige Identifizierung des Werkzeugs durch Auslesen des Datenträgers.

Unter einem Werkzeug im Sinne der Erfindung ist insbesondere ein chirurgisches Werkzeug / Operationswerkzeug mit einem in der Regel distalen, scharfkantigen Wirkabschnitt zum Erfüllen einer operativen Funktion und einem proximalen Koppelabschnitt zum Koppeln des Werkzeugs mit einer Griffeinheit, insbesondere einer Antriebsgriffeinheit, zu verstehen. Beispiele für solche Werkzeuge sind vor allem rotierende Werkzeuge sowie Sägewerkzeuge, unter anderem Bohrer, Fräser, Sägen, Schraubaufsätze, Schneidklingen oder Schleifaufsätze, die in allgemein bekannter Weise mit einer Griff- und/oder Antriebseinheit gekoppelt werden. Außerdem ist unter einem Werkzeug im Sinne der Erfindung eine Sprühdüse, eine HF-Spritze, eine Ultraschallklinge, etc. zu verstehen. Schließlich beinhaltet der Begriff "Werkzeug" im Sinne der Erfindung auch Implantate beliebiger Art und Form, wie zum Beispiel Knochen- und Gelenkimplantate oder -teilimplantate, Stents, etc.

Es ist ein besonderer Vorteil der Erfindung, dass das Werkzeug bei einer anwenderseitigen Entnahme aus der Verpackung automatisch erkannt werden kann. Der Datenträger ist zur vorzugsweise drahtlosen Kommunikation mit externen Leseund/oder Schreibeinheit ausgebildet. Besonders vorteilhaft ist, wenn ein mit dem Werkzeug zu verwendendes Griffstück eine Ausleseeinrichtung aufweist, mittels der die auf dem Datenträger enthaltenen Daten erkannt und/oder ausgelesen werden können, da dann das Werkzeug automatisch beim Verbinden / Anordnen / Stecken mit dem Griffstück identifiziert werden kann. Derart erfasste Daten können dem Anwender auf einer mit dem Griffstück kommunizierenden Displayeinheit übermittelt / angezeigt werden.

Die auf den Datenträger geschriebenen Daten können im Sinne der Erfindung sein eine Artikelnummer, eine Losnummer, eine Chargennummer, ein Mindesthaltbarkeitsdatum, ein Verfallsdatum oder maximales Verwendungsdatum, Material, Abmessungen und Geometrien, Verwendungszweck, bisherige Verwendungen und Verwendungsdauer, Lagerbestände, etc.

Die allgemeine der Erfindung zugrundeliegende Idee beinhaltet, dass eine Werkzeugerkennung stattfindet und durch Datenübertragung an Peripheriegeräte weitergegeben wird und dem Endkunden ausgewählte Daten angezeigt werden. Gleichzeitig können weitere Daten geloggt und weiterverarbeitet werden.

Vorteilhafte Ausführungsformen der Erfindung sind in den Unteransprüchen beansprucht und werden nachfolgend näher erläutert.

Eine Ausführungsform der Erfindung ist dadurch gekennzeichnet, dass die Haltevorrichtung lagebestimmt in dem Aufnahmeraum angeordnet und/oder gehalten ist. Sie dient insbesondere dazu, das Werkzeug zu halten, insbesondere lagebestimmt zu halten und im Aufnahmeraum derart zu positionieren, dass ein Kontakt von scharfen Wirkabschnitten mit dem Verpackungsmaterial vermieden wird und scharfe Wirkabschnitte von der Verpackung beabstandet sind. Durch Vorsehen einer solchen Haltevorrichtung kann ein Kontakt des Werkzeugs mit Teilen der Verpackung verhindert oder zumindest sicher minimiert werden, so dass Abrieb von Verpackungsmaterial durch das Werkzeug verhindert werden kann. Die Haltevorrichtung und/oder die Umverpackung können zumindest teilweise, vorzugsweise vollständig, aus einem resorbierbaren Material bestehen. Der Aufnahmeraum kann durch die Umverpackung geschlossen ausgebildet, insbesondere hermetisch dicht sein. Er kann außerdem steril ausgebildet sein. Ein besonderer Vorteil der Erfindung ist, dass der Datenträger ausgelesen werden kann, ohne dass die Verpackung geöffnet und die Sterilität aufgegeben werden muss.

Nach der Erfindung ist die Haltevorrichtung separat von der Umverpackung ausgebildet. Daher kann das in der Haltevorrichtung gehaltene Werkzeug zusammen mit dieser aus der Umverpackung entnommen werden und insbesondere auch nach dem Auspacken bis zum letztlichen Gebrauch in der Haltevorrichtung verbleiben. Vorzugsweise sind Verpackung und Haltevorrichtung derart ausgebildet, dass ein Entnehmen durch Handhabung der Haltevorrichtung und ohne direkte Kontaktierung des Werkzeugs erfolgen kann, so dass die Wahrscheinlichkeit einer Kontaminierung des Werkzeugs einfach und sicher minimiert, wenn nicht sogar vermieden werden kann. Außerdem kann das Werkzeug zusammen mit der Haltevorrichtung auf einem Instrumentiertisch platziert werden, ohne es lose ablegen zu müssen. Während einer Operation oder Behandlung kann die Halterung in besonders vorteilhafter Weise zur Zwischenlagerung des Werkzeugs verwendet werden, was zu besserer Ordnung und Übersichtlichkeit führt. Die Erfindung kann damit neben der Verpackung im eigentlichen Sinne durch die separate Haltevorrichtung eine eigenständige Halterung zur Verfügung stellen, die neben einer sicheren und stabilen Lagerung von Werkzeugen während des Transports in der jeweiligen Umverpackung außerdem eine Möglichkeit zur definierten Positionierung und sicheren Handhabung des Werkzeugs im Rahmen einer Aufbereitung (z.B. Sterilisierung) sowie bei einer Bereitstellung des Produkts während einer Behandlung im OP ermöglicht. Dazu kann die Haltevorrichtung zusammen mit dem darin gehaltenen Werkzeug aus der Umverpackung genommen und aufgestellt werden.

Der Datenträger kann nach einer Ausführungsform der Erfindung ein RFID-Tag oder ein NFC-Tag sein. Dabei ist es besonders vorteilhaft, dass seine Daten berührungslos und automatisch ausgelesen, geschrieben und verarbeitet werden können, insbesondere jederzeit, ohne dass es dazu einer besonderen Interaktion des Anwenders bedarf. Insbesondere kann der Datenträger permanent auslesbar sein. Auch kann sowohl an der Umverpackung wie auch an der Haltevorrichtung ein Datenträger angeordnet sein. Der Datenträger kann insbesondere aufgeklebt sein.

Nach einer Ausführungsform der Erfindung sind der Datenträger und die Umverpackung bzw. der Datenträger und die Haltevorrichtung miteinander fest oder unlösbar verbunden. Vorzugsweise ist der Datenträger damit verklebt, vergossen oder verpresst. Infolge dessen ist ein versehentliches Lösen des Datenträgers sowie eine ungewollte Separation von Datenträger und Verpackung / Werkzeug ausgeschlossen, so dass mit großer Sicherheit gewährleistet ist, dass die auf dem Datenträger vorliegenden Daten wirklich zu dem in der Verpackung / in der Haltevorrichtung befindlichen Werkzeug gehören. Dies erhöht in vorteilhafter Weise die Patientensicherheit.

Nach einer weiteren Ausführungsform der Erfindung ist der Datenträger aktivierbar. Eine Aktivierung des Datenträgers kann insbesondere durch eine Relativpositionierung des Werkzeugs relativ zu der Verpackung bzw. der Umverpackung bzw. der Haltevorrichtung erfolgen. Zum Beispiel kann durch eine derartige Relativpositionierung ein mit dem Datenträger verbundener Antennenkreis und/oder Stromkreis und/oder Schaltkreis geschlossen/ aktiviert werden, wodurch der Datenträger aktiviert wird und ausgelesen werden kann. Dies kann dadurch bewirkt werden, dass bei in bestimmungsgemäßer Weise in der Verpackung positioniertem Werkzeug der Stromkreis und/oder Antennenkreis und/oder Schaltkreis offen ist, so dass er inaktiv ist, zum Beispiel da das Werkzeug selbst isolierend ist und den Kreis unterbricht, oder da das Werkzeug die Verpackung / Umverpackung / Haltevorrichtung derart verformt, dass der Kreis unterbrochen ist. Verfahrensgemäß wird der Datenträger durch eine Relativpositionierung des Produkts in der Aufnahme bzw. in der Haltevorrichtung oder durch eine Entnahme daraus für ein Auslesen der auf dem Datenträger befindlichen Daten oder ein Senden der auf dem Datenträger befindlichen Daten aktiviert.

Der Antennen- und/oder Stromkreis kann Bestandteil der Verpackung sein. Er kann insbesondere in die Umverpackung und/oder die Haltevorrichtung integriert oder daran angeordnet oder ausgebildet sein. Vorzugsweise ist der Kreis derart ausgebildet, dass er bei in bestimmungsgemäßer Weise in der Verpackung angeordnetem Werkzeug offen ist, also deaktiviert ist, während er bei aus der bestimmungsgemäßen Lage entfernten Werkzeug geschlossen wird bzw. ist, also aktiviert ist. Dadurch wird der Vorteil erzielt, dass der Datenträger erst im Rahmen einer Entnahme des Werkzeugs aus der Verpackung ausgelesen wird, so dass unnötiger Datenverkehr und Energieverbrauch vermieden oder verringert werden kann. Im Hinblick auf das erfindungsgemäße Verfahren kann der Datenträger beim Einbringen des in der Verpackung befindlichen Werkzeugs, insbesondere des in der Haltevorrichtung befindlichen Werkzeugs, in ein zur Aufnahme des Werkzeugs bestimmtes Handstück durch eine vorzugsweise in dem Handstück befindliche Leseeinrichtung ausgelesen werden.

Verfahrensgemäß kann das Werkzeug in der Haltevorrichtung angeordnet werden und der Datenträger vor oder nach Anordnen des Werkzeugs in der Haltevorrichtung vorzugsweise unter Sterilbedingungen beschrieben werden. Dies ermöglicht eine große Flexibilität. Eine Ausführungsform des erfindungsgemäßen Verfahrens sieht vor, dass nach dem Verschließen der Verpackung eine γ-Sterilisation durchgeführt wird. Dadurch kann Sterilität bewirkt werden, ohne die sichere Funktion des Datenträgers zu gefährden. Das Beschreiben des Datenträgers kann nach einer Ausführungsform auch nach dem Durchführen der Sterilisation durchgeführt werden.

Es ist von besonderem Vorteil, wenn nach einer weiteren Ausführungsform der Erfindung aus dem Datenträger ausgelesene Daten einem Anwender des Werkzeugs dargestellt werden. Alternativ oder zusätzlich können aus dem Datenträger ausgelesene Daten des Werkzeugs gespeichert und/oder an den Anbieter des Werkzeugs geleitet werden. Die genannten Vorgänge laufen vorzugsweise automatisch ab, so dass die Daten insbesondere automatisch dargestellt und/oder weitergeleitet und/oder in einer Datenbank gespeichert werden können.

Die Haltevorrichtung kann eine Grundplatte aufweisen. Diese kann insbesondere derart ausgebildet sein, dass sie eine Standfläche zum lagestabilen Aufstellen der Haltevorrichtung mit einem darin gehaltenen Werkzeugs, insbesondere in einem aus der Verpackung ausgepackten Zustand, und/oder eine Anlagestruktur zur Anlage an der Verpackung ausbildet. Neben der Standfläche kann die Haltevorrichtung weitere Anlagestrukturen oder -abschnitte aufweisen, die mit der Verpackung zusammenwirken und an dieser anliegen, so dass eine eindeutige und stabile Lagepositionierung in der Aufnahme der Verpackung gewährleistet ist. Wenn der Datenträger im Rahmen der Erfindung in eine solche Haltevorrichtung integriert oder daran angeordnet ist, ist eine Identifizierung des in der Haltevorrichtung zum Beispiel auf einem Instrumentierungstisch abgestellten Werkzeugs besonders einfach im Rahmen eines medizinischen Eingriffs möglich.

Die Haltevorrichtung kann nach einer Ausführungsform der Erfindung zumindest zwei voneinander beabstandete Haltestrukturen zum lagebestimmten Positionieren des Werkzeugs aufweisen. Bevorzugt sind diese jeweils an der Grundplatte angeordnet und können sich von dieser in den Aufnahmeraum hinein erstrecken. Neben der Funktion des Haltens des Werkzeugs können die Haltestrukturen Anlagen ausbilden, mit denen die Haltevorrichtung an der Verpackung anliegt, vorzugsweise derart, dass sie in der Aufnahme der Verpackung lagegesichert oder lagefixiert ist. Eine der Haltestrukturen kann endseitig an der Haltevorrichtung, insbesondere an der Grundplatte, angeordnet sein. Eine andere der Haltestrukturen kann im Wesentlichen mittig an der Haltevorrichtung, insbesondere an der Grundplatte, angeordnet sein. Dies ermöglicht eine besonders stabile Halterung des Werkzeugs in der Haltevorrichtung.

Vorzugsweise ist eine der Haltestrukturen zum lagebestimmten Positionieren des Werkzeugs relativ zur Haltevorrichtung in einer ersten Richtung ausgebildet. Die andere der Haltestrukturen kann zum lagebestimmten Positionieren des Werkzeugs relativ zur Haltevorrichtung in einer zweiten Richtung und einer dritten Richtung ausgebildet sein, wobei die erste, zweite und dritte Richtung zueinander orthogonal sind. Durch eine derartige Führung ist das Werkzeug einerseits stabil in der Haltevorrichtung gehalten, andererseits kann es besonders einfach durch einen Anwender in der Haltevorrichtung angeordnet und aus der Haltevorrichtung entnommen werden. Es ist besonders vorteilhaft, wenn nach einer weiteren Ausführungsform der Verpackung zumindest eine der Haltestrukturen mit dem Werkzeug einen Formschluss und/oder einen Kraftschluss ausbildet, insbesondere mit einer Koppelstruktur des Werkzeugs.

Zumindest eine der Haltestrukturen, insbesondere beide Haltestrukturen, kann bzw. können jeweils zwei einander gegenüberliegende Haltearme aufweisen. Zwischen diesen ist jeweils ein Schlitz zur Aufnahme und Halterung des Werkzeugs ausgebildet. Die Haltearme können im Wesentlichen senkrecht zur Grundplatte der Haltevorrichtung angeordnet sein. Sie können in besonders vorteilhafter Weise als Klemmarme ausgebildet sein und insbesondere in einer Richtung quer zum Schlitz federnde Eigenschaften aufweisen. Ein Entnehmen sowie ein Anordnen des Werkzeugs in den Haltestrukturen ist besonders einfach möglich, wenn nach einer Form der Erfindung sich der Schlitz von proximal der Basis in Richtung distal der Basis aufweitet. Dies begünstigt eine weitgehend gefahrlose Handhabung sowohl von ungebrauchten als auch von gebrauchten Instrumenten, so dass insbesondere eine verletzungsfreie Entsorgung von Werkzeugen in der Halterung verbessert und die dabei grundsätzlich bestehende Infektionsgefahr verringert wird. Nach einer Fortbildung der Erfindung können die dem Schlitz zugewandten Seiten der Haltearme mit Einbuchtungen versehen sein. Solche Einbuchtung können in vorteilhafter Weise Rastbuchten für das in den Schlitz eingebrachte Werkzeug bilden, so dass dieses sicher und dennoch für einen Nutzer einfach entnehmbar in den Haltestrukturen gehalten ist.

Eine besonders nutzerfreundliche, da Verletzungen eines Nutzers an dem in der Haltevorrichtung vermeidende Ausführungsform der Erfindung sieht vor, dass die Haltevorrichtung eine Schutzlasche aufweist, die von der Grundplatte in den Aufnahmeraum hineinragt und ein distales Ende des Werkzeugs oder eine scharfkantige Struktur des Werkzeugs berührungslos abdeckt. Auf diese Weise ist ein Anwender beim Handhaben der Haltevorrichtung mit dem darin gehaltenen Werkzeug vor Verletzungen durch gegebenenfalls scharfe Kanten des Werkzeugs geschützt.

Gemäß der Erfindung ist die Verpackung als Blister ausgebildet. Solche Blister sind günstig und können für nahezu beliebige Formen von Produkt und/oder Haltevorrichtung hergestellt werden. Die Blisterverpackung weist eine Unterschale mit einer den Aufnahmeraum für das Werkzeug ausbildenden Vertiefung auf. Eine solche Unterschale kann einfach mit einer zur lagestabilen Positionierung von Haltevorrichtung und Werkzeug ausreichenden Stabilität und Form ausgebildet sein. Durch eine an der Unterschale in bekannter Weise angeordnete Deckelfolie kann der Aufnahmeraum in der jeweils gewünschten Weise verschlossen sein, insbesondere hermetisch und/oder steril. Der Datenträger kann an der Unterschale oder an der Deckelfolie positioniert sein.

Nach einer weiteren Ausführungsform der Erfindung kann die Verpackung ein Trägerelement zum Beispiel nach Art eines Siebkorbs aufweisen. Dieses kann zusammen mit der Haltevorrichtung und dem daran gehaltenen Werkzeug, insbesondere mit einer Mehrzahl an Haltevorrichtungen mit dem jeweils daran gehaltenen Werkzeug, in der Verpackung angeordnet sein. Mittels eines solchen Trägerelements können mehrere Haltevorrichtung mit darin gehaltenen Werkzeugen besonders einfach und gleichzeitig gehandhabt werden, zum Beispiel einer Sterilisierung zugeführt und/oder auf einem Instrumentierungstisch abgestellt werden.

Nach einer weiteren Ausführungsform der Erfindung kann die Haltevorrichtung kodiert sein, beispielsweise indem sie farbig ausgebildet ist. Auf diese Weise kann mit der Erfindung besonders einfach ein System bereitgestellt werden, mit dem nicht nur unterschiedliche Produkte für einen Nutzer einfach unterscheidbar kodiert sein können, sondern darüber hinaus eine solche Kodierung oder Kennzeichnung bestimmter Werkzeuge auch nach den Entpacken des Werkzeugs aus der Verpackung dem jeweiligen Werkzeug zugeordnet beibehalten werden kann. Insbesondere sind so bereits entpackte und für eine Verwendung auf einem Instrumentierungstisch oder in einer Sterilisationseinrichtung befindliche Werkzeuge für den Anwender besonders einfach und sicher zu erkennen. Eine Identifikation unterschiedlicher Werkzeuge und/oder Haltevorrichtung kann vorzugsweise über unterschiedliche Farbgebungen der Haltevorrichtungen erfolgen, beispielsweise im Hinblick auf deren Indikation oder auf bestimmte Produktgruppen, etc. Auf diese Weise können Fehlkombinationen von Werkzeugen durch die Farbgebung der insbesondere als Kunststoffspritzteil ausgebildeten Haltevorrichtung in und außerhalb der Verpackung vermieden werden.

Nach einer weiteren Ausführungsform der Erfindung kann die Haltevorrichtung als Kunststoffformteil ausgebildet sein. Wenn sie nach einer Fortbildung der Erfindung als Mehrkomponentenspritzguss ausgebildet ist, kann es zum Beispiel mit einer besonders rutschfesten Standfläche versehen sein. Dies ermöglicht eine stabile Anordnung der Haltevorrichtung auf einem Instrumentiertisch und/oder einem Trägerelement wie einem Siebkorb während eines Aufbereitungsprozesses. Außerdem kann so die Handhabbarkeit der Haltevorrichtung bei der Entnahme des ungebrauchten Werkzeugs wie auch bei der Einführung eines gebrauchten Werkzeugs ungemein erleichtern werden. Der Datenträger kann insbesondere in die Haltevorrichtung eingeformt / eingespritzt sein.

Es ist ein besonderer Vorteil der Erfindung, dass ein Nutzer im Rahmen einer Verwendung eines bestimmten medizintechnischen Werkzeugs sicher, einfach, schnell und insbesondere automatisch Informationen darüber erhalten kann, welches Werkzeug er gerade nutzt oder zu nutzen er beabsichtigt und/oder auf eine Griffeinheit gesteckt ist/wird, ohne dazu extra ein Label oder die Umverpackung sichten zu müssen. Insbesondere kann ein Anwender automatisch und einfach erkennen, ob das verwendete Produkt für eine betreffende Anwendung geeignet oder ungeeignet ist. Ein weiterer Vorteil der Erfindung ist, dass ein Anwender einfach und ohne dazu eine Inventur durchführen zu müssen, welche Bestände des betreffenden Werkzeugs sich noch an seinem Lager (ggf. Konsignationslager) befinden.

Die vorliegende Erfindung bietet nicht nur für den Anwender, sondern auch für den Anbieter von verpackten medizintechnischen Produkten und Werkzeugen wesentliche Vorteile: Er ist durch die Erfindung in der Lage, besonders einfach zu ermitteln, welche Produkte / Werkzeuge kombiniert und/oder verwendet wurden. Außerdem kann er eine Überlastung von Werkzeugen und damit gegebenenfalls verbundene Produktschäden nachvollziehen. Schließlich wird es für einen Anbieter durch die Erfindung ermöglicht, einem Kunden eine angepasste sozusagen maßgeschneiderte Logistik anzubieten.

Zusammenfassend kann man sagen, dass die Erfindung eine Erkennung, insbesondere eine automatische Erkennung, des jeweilig verwendeten Produkts, insbesondere des jeweils auf einen Handgriff oder Antriebseinheit gesteckten Werkzeugs, ermöglicht. Außerdem ermöglicht die Erfindung eine besonders einfache und weitgehend fehlersichere Pflege und/oder Übermittlung von auf den Datenträger geschriebenen beliebigen produktbezogenen Daten. Die Erfindung ermöglicht insbesondere die folgenden Vorteile:
- Direkte, automatisierte Produkterkennung sowohl für einen Nutzer als auch für einen Anbieter (Supply Chain Management (SCM), Service, Fehleranalyse)
- Übermittlung von beliebig vielen weiteren Daten an den Nutzer und/oder den Anbieter (Supply Chain Management (SCM), Service, Fehleranalyse)
- für den Nutzer schafft die Erfindung eine Möglichkeit, produktbezogene Daten automatisiert und zeitnah in einer Patientenakte zu vermerken.

In anderen Worten ausgedrückt stellt die vorliegende Erfindung eine Werkzeugaufnahme oder ein Werkzeugauflager bereit, das dazu angepasst und vorgesehen ist, in einer Sterilverpackung aufgenommen zu werden oder darin ausgebildet zu sein, um einen Bestandteil der Verpackung zu bilden. Das Werkzeugauflager hat zumindest eine, vorzugsweise mehrere (axial-) beabstandete Schaftaufnahmeklemmen, (jeweils bestehend aus zwei federelastischen oder federelastisch gelagerten Klemmarmen / Klemmbacken) die an einer Sockel- oder Grundplatte vorzugsweise stoffeinstückig angeordnet ist/sind und bevorzugt jeweils zumindest eine oder mehrere Ausbauchungsabschnitte zur (teilweisen) umgreifenden Aufnahme eines Werkzeugschafts ausbildet/ausbilden.

Zur Vermeidung einer Axialbewegung des Werkzeugs/Werkzeugschafts in der/den Werkzeugaufnahmeklemme(n) sind zwei (axial-) beabstandete Endanschläge an der Grundplatte vorgesehen/montierbar.

Der auslesbare Datenträger (Tag) kann insbesondere an/in der Grundplatte axial zwischen den zwei Endanschlägen und vorzugsweise axial zwischen zwei Schaftauflageklemmen platziert sein.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 eine perspektivische Ansicht einer Ausführungsform einer Verpackung nach der Erfindung,
Fig. 2 eine perspektivische Ansicht einer Ausführungsform einer Verpackung nach der Erfindung,
Fig. 3 eine perspektivische Ansicht einer Haltevorrichtung mit darin gehaltenem Werkzeug,
Fig. 4 eine perspektivische Ansicht einer Haltevorrichtung mit darin gehaltenem Werkzeug,
Fig. 5 ein vergrößertes Detail der Fig. 4,
Fig. 6 eine Mehrzahl an Haltevorrichtungen mit jeweils einen darin gehaltenen Werkzeug,
Fig. 7 eine Schnittansicht einer Haltevorrichtung durch deren Haltestruktur quer zur Längsachse,
Fig. 8 eine Schnittansicht einer Haltevorrichtung in Richtung der Längsachse,
Fig. 9 eine Aufsicht auf die Haltevorrichtung der Figur 2 und
Fig. 10 eine Schnittansicht der Haltevorrichtung der Figur 9.

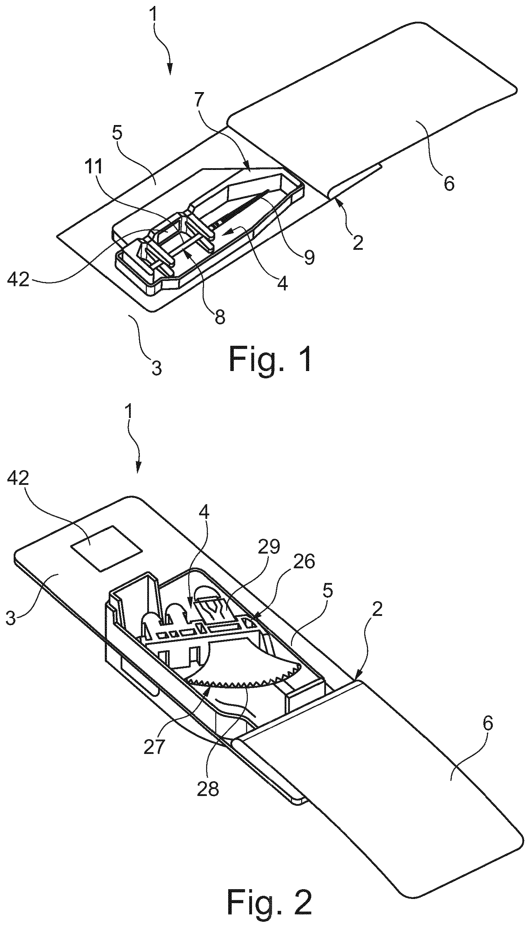

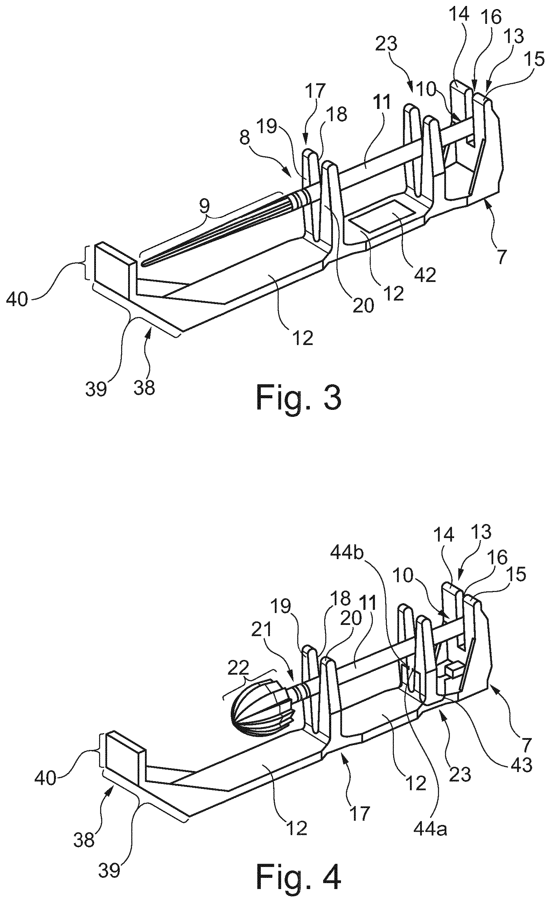

Die Figuren 1 und 2 zeigen jeweils eine Ausführungsform einer Verpackung 1 nach der Erfindung. Diese umfasst eine Umverpackung 2 in Form eines Blisters 2 mit einer Unterschale 3 mit einer darin ausgebildeten Vertiefung 4 und einem die Vertiefung 4 umgebenden Rand 5. Die Umverpackung 2 umfasst außerdem eine an dem umlaufenden Rand 5 der Unterschale 3 angeordnete und die Vertiefung 4 hermetisch dicht verschließende Deckelfolie 6, die in den Figuren 1 und 3 jeweils in einem teilweise geöffneten Zustand gezeigt ist. Sowohl die Unterschale 3 wie auch die Deckelfolie 6 können aus einem resorbierbaren Material bestehen.

In der Vertiefung 4 der Umverpackung 2 der Figur 1 ist eine Haltevorrichtung 7 mit einem darin gehaltenen Werkzeug 8 als Beispiel für ein verpacktes Produkt angeordnet (siehe auch Figur 3). Das Werkzeug 8 ist im vorliegenden Ausführungsbeispiel ein Bohrer 8, an dessen distalen Ende ein Wirkabschnitt 9 in Form eines Bohrkopfes 9 und an dessen proximalen Ende eine Koppelstruktur 10 zum Anordnen des Werkzeugs 8 in einer nicht dargestellten Werkzeugaufnahme einer Antriebsgriffeinheit ausgebildet sind. Zwischen dem Wirkabschnitt 9 und der Koppelstruktur 10 weist das Werkzeug 8 einen langgestreckten Werkzeugschaft 11 auf.

Erfindungsgemäß ist an der Haltevorrichtung 7 der in Figur 1 gezeigten Ausführungsform ein Datenträger 42, hier in Form eines über Funk auslesbaren / beschreibbaren Datenträgers, zum Beispiel in Form eines NFC- oder RFID-Tags 42, angeordnet. In der Ausführungsform der Figur 2 ist ein Datenträger 42, auch hier in Form eines RFID-Tags 42, an der Umverpackung 2 angeordnet. Auf den Datenträgern 42 der Figuren 1 und 2 sind für das jeweilige Werkzeug 8 spezifische Daten gespeichert.

Die Haltevorrichtung 7 der Ausführungsform der Figur 1 ist in Figur 3 ohne die Umverpackung 2 gezeigt. Sie ist als Kunststoffformteil ausgebildet, besteht vollständig aus einem resorbierbaren Material und weist eine Grundplatte 12 auf. Am proximalen Ende der Grundplatte 12 ist eine erste Haltestruktur 13 ausgebildet. Diese umfasst zwei zueinander parallele, sich von der Grundplatte 12 im Wesentlichen jeweils orthogonal in Richtung des Werkzeugs 8 erstreckende und mit der Grundplatte 12 verbundene Haltearme 14, 15. Zwischen den Haltearmen 14, 15 ist ein Schlitz 16 ausgebildet, als Aufnahme für die Koppelstruktur 10 des Werkzeugs 8. Mittig der Grundplatte 12 ist eine zweite Haltestruktur 17 ausgebildet, die sich von der Grundplatte 12 in die gleiche Richtung erstreckt wie die erste Haltestruktur 13. Die zweite Haltestruktur 17 besitzt ebenfalls zwei einander gegenüberliegende und zwischen sich einen Aufnahmeschlitz 18 ausbildende Haltearme 19, 20, die beide zur Grundplatte 12 im Wesentlichen orthogonal angeordnet sind. Zwischen der ersten Haltestruktur 13 und der zweiten Haltestruktur 17 ist eine dritte Haltestruktur 23 ausgebildet, die im Wesentlichen der zweiten Haltestruktur 17 gleicht und daher nicht weiter beschrieben wird. Der Datenträger 42 ist auf der dem Werkzeug 8 zugewandten Seite der Grundplatte 12 angeordnet, hier aufgeklebt.

Figur 4 zeigt eine zur Haltevorrichtung 7 der Figur 3 ähnliche Haltevorrichtung 7 mit einem anderen Werkzeug 21, hier in Form eines Fräsaufsatzes 21, sowie einem anderen Datenträger 42 in Form eines über Funk auslesbaren / beschreibbaren Datenträgers, zum Beispiel in Form eines NFC- oder NFC-Tags 42. Der Fräsaufsatz 21 weist wie der Bohrer 8 der Figuren 1 und 3 eine Koppelstruktur 10 und einen Werkzeugschaft 11 auf und unterscheidet sich vom Bohrer 8 durch seinen als Fräskopf 22 ausgebildeten distalen Wirkabschnitt 22. Dennoch können durch die Erfindung der Bohrer 8 und der Fräskopf 21 in gleichen Haltevorrichtungen 7 aufgenommen werden. Der Datenträger 42 ist wiederum an der Grundplatte 12 angeordnet, und zwar nahe der Koppelstruktur 10 des Fräseraufsatzes 21. Er ist mit einem Antennenkreis 43 verbunden. Dieser weist zwei Kontakte 44a und 44b auf, die bei in Figur 4 bestimmungsgemäß in der Haltevorrichtung 7 angeordnetem Werkzeug 21 elektrisch voneinander getrennt sind, so dass der Antennenkreis 43 unterbrochen ist. Der Antennenkreis kann sich nach einer weiteren Ausführungsform auf der Unterseite der Haltevorrichtung 7 befinden und recht groß ausgestaltet sein. Der angedeutete Stromkreisschluss soll symbolisieren, dass dort die Funktion der Antenne dann aktiviert wird. Ebenso kann der Stromkreisschluss durch andere konstruktive Ausgestaltungen, zum Beispiel durch Blattfedern usw. bewerkstelligt werden. Die Grundidee jedoch ist, dass immer ein Stromkreis bei der Entnahme des WZGs geschlossen oder geöffnet wird.

In der in Figur 4 gezeigten Ausführungsform sind die zwei Kontakte 44a und 44b im Bereich der dritten Haltestruktur 23 vorgesehen. Es ist jedoch auch denkbar, dass die Kontakte 44a und 44b in unterschiedlichen Bereichen der Haltevorrichtung 7 vorgesehen sind. Beispielsweise kann der Kontakt 44a im Bereich der/ an der dritten Haltestruktur 23 und der Kontakt 44b im Bereich der/ an der ersten Haltestruktur 13 vorgesehen/ angeordnet sein. Wenn das Werkzeug 21 in der Haltevorrichtung 7 angeordnet ist, ist bevorzugt auch in diesem Fall der Antennenkreis 43 unterbrochen/ geschlossen. Wenn die Kontakte 44a und 44b in unterschiedlichen Bereichen der Haltevorrichtung 7 vorgesehen sind, kann eine ungewollte Kontaktierung ohne Werkzeug besser vermieden werden.

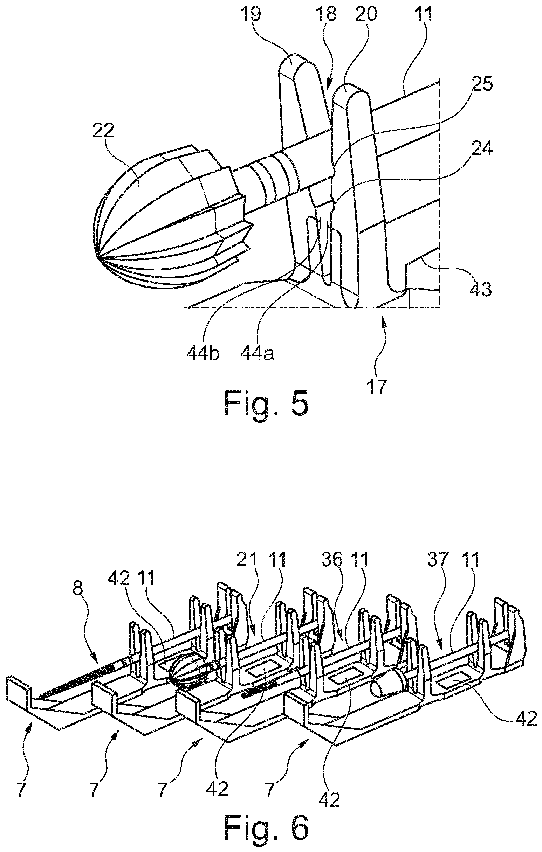

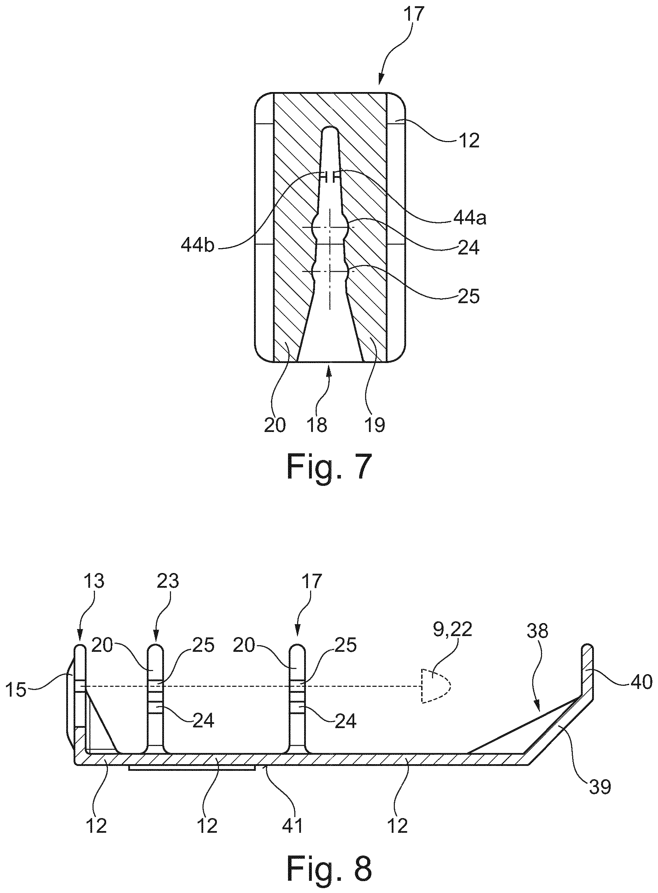

Figur 5 zeigt die zweite Haltestruktur 17 der Haltevorrichtung 7 in einer vergrößerten Detailansicht. Die einander zugewandten Seitenflächen der Haltearme 19, 20 sind mit jeweils zwei übereinander angeordneten Einbuchtungen 24, 25 von unterschiedlicher Größe versehen, jeweils zur Aufnahme von Werkzeugschäften 11 unterschiedlichen Durchmessers (siehe auch Figur 7, die die zweite Haltestruktur 17 in einer Schnittansicht quer zur Längsachse des Werkzeugs 8, 21 zeigt). Die Haltearme 19, 20 weisen gewisse elastische Federeigenschaften auf und können relativ zueinander eine Federbewegung ausführen, so dass sich der Schlitz 18 bei einem Einbringen eines Werkzeugs 8, 21 aufweiten kann und die Haltearme 19, 20 in ihre ursprüngliche Position zurückfedern, sobald der Schaft 11 des entsprechenden Werkzeugs 8, 21 in der Einbuchtung 24, 25 angeordnet ist. Auf diese Weise ist das Werkzeug 8, 21 sicher in der Haltevorrichtung 7 gehalten. Es kann insbesondere zwischen den Haltearmen 19, 20 geklemmt gehalten sein. Bei in der Einbuchtung befindlichem Werkzeug 8, 21 sind die Halterarme 19, 20 jedoch nicht wieder vollständig in ihre ursprüngliche Lage zurückgefedert, sondern sind immer noch um ein gewisses Maß weiter voneinander beabstandet, so dass die beiden Kontakte 44a, 44b des mit dem Datenträger elektrisch verbundenen Antennenkreises 43 voneinander getrennt sind und der Antennenkreis 43 unterbrochen ist. Erst wenn das Werkzeug 8, 21 aus dem zwischen den beiden Haltearmen 19, 20 befindlichen Schlitz 18 entfernt ist, können die Haltearme 19, 20 wieder vollständig in ihre ursprüngliche Lage zurückfedern, in der beiden Kontakte 44a, 44b miteinander in Kontakt stehen, so dass bei entferntem Werkzeug 8, 21 der Antennenkreis 43 geschossen ist und der damit elektrisch verbundene Datenträger 42 ausgelesen werden kann.

An dem der ersten Haltestruktur 13 gegenüberliegenden (wirkabschnittseitigen) Ende der Grundplatte 12 ist an dieser eine Schutzlasche 38 angeordnet. Die Schutzlasche 38 ragt von der Grundplatte 12 zunächst mit einem Schrägabschnitt 39 in einem schrägen Winkel (hier von etwa 45°) ab und ist dann um einen weiteren Winkel zu einem Endabschnitt 40 gebogen, so dass ihr von der Grundplatte 12 abgewandtes Ende in etwa quer zu dieser angeordnet ist. Wie insbesondere Figur 8 zeigt, ragt die Schutzlasche 38 derart weit von der Grundplatte 12 ab, dass das jeweilige distale Ende des Wirkabschnitts 9, 22 berührungsfrei abgedeckt ist. Diese Ausgestaltung der Schutzlasche 38 schafft eine gute Griffmöglichkeit in Form des Schrägabschnitts 39 zum Ergreifen durch einen Nutzer, zum Beispiel aus der in Figur 6 gezeigten Position von einem Instrumentierungstisch, wobei der Wirkabschnitt verdeckt ist, so dass dessen Kontaktierung durch den Nutzer sicher verhindert und damit ein Verletzungsrisiko minimiert werden kann. Figur 8 zeigt außerdem ein auf die Unterseite der Grundplatte 12 aufgeklebten über Funk auslesbaren / beschreibbaren Datenträger, zum Beispiel in Form eines NFC- oder RFID-Tags 42.

Figur 6 zeigt, dass die Haltevorrichtung 7 zur Aufnahme und zum Halten von unterschiedlichen Werkzeugen 8, 21 geeignet ist, solange diese eine im Wesentlichen ähnliche Grundform mit einem Schaft 11 und einer Koppelstruktur 10 am proximalen Ende aufweisen und sich eigentlich nur hinsichtlich ihres jeweiligen Wirkabschnitts 9, 22 unterscheiden. Beispielhaft gezeigt sind in Figur 6 der Bohraufsatz 8, der Fräsaufsatz 21, ein Schraubaufsatz 36 und ein Schleifaufsatz 37, wobei die Schaftdurchmesser der Aufsätze 36, 37 kleiner sind als die der Aufsätze 8, 21, so dass die Aufsätze 36, 37 in der Einbuchtung 24 mit kleinerem Durchmesser und die Aufsätze 8, 21 in der Einbuchtung 25 mit größerem Durchmesser angeordnet sind. Des Weiteren ergibt sich aus Figur 6 wie auch aus Figur 3 die Eignung der Haltevorrichtung 7 (gleiches gilt für die im Folgenden beschriebene Haltevorrichtung 26) neben der Lagefixierung des Werkzeugs 8, 21 im verpackten Zustand, also in dem in den Figuren 1 und 2 gezeigten Zustand, als separat von der Umverpackung 2 nutzbare Haltevorrichtung genutzt zu werden, um das Werkzeug abzustellen, beispielsweise bei einer Bereitstellung im Rahmen einer OP oder bei einer Sterilisierung. Die Unterseite 41 der Grundplatte 12, 30 bildet dazu eine Standfläche 41, auf der die Haltevorrichtung 7, 26 mit einem darin gehaltenen Werkzeug stabil abstellbar ist. Schließlich zeigt Figur 6, dass die beiden Kontakte 44a und 44b des Antennenkreises 43 miteinander in Kontakt stehen, wenn sich kein Werkzeug 8, 21 in der Haltevorrichtung 7 befindet. Figur 5 zeigt eine Beabstandung und würde sich bei Entfernung des Werkzeuges verbinden.

In der Vertiefung 4 der Umverpackung 2 der Figur 2 ist eine Haltevorrichtung 26 mit einem darin gehaltenen Werkzeug 27 angeordnet. Das Werkzeug 27 ist im vorliegenden Ausführungsbeispiel ein Sägeaufsatz 27, an dessen distalen Ende ein Wirkabschnitt 28 in Form eines Sägeblatts und an dessen proximalen Ende eine Koppelstruktur 29 zum Anordnen des Werkzeugs 27 in einer nicht dargestellten Werkzeugaufnahme einer Antriebsgriffeinheit ausgebildet sind.

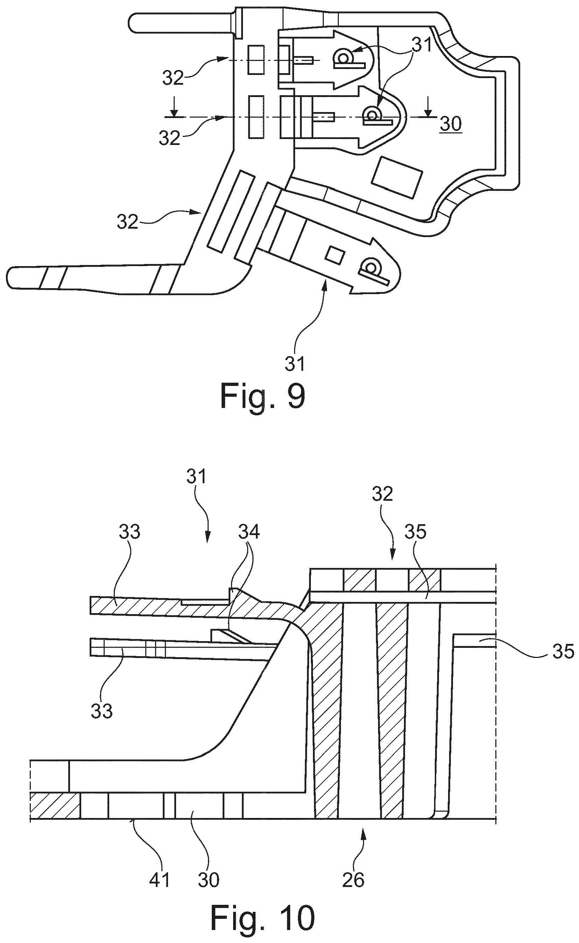

Wie die Figuren 9 und 10 zeigen, weist die Haltevorrichtung 26 eine Grundplatte 30 auf, auf deren Oberseite ein über Funk auslesbarer / beschreibbarer Datenträger, zum Beispiel in Form eines NFC- oder RFID-Tags 42 als Datenträger mit werkzeugspezifischen Daten angebracht ist. An der Grundplatte 30 sind außerdem eine erste Haltestruktur 31 und eine zweite Haltestruktur 32 angeordnet, die in einer Richtung quer zur Grundplatte 30 von dieser beabstandet sind. Die Haltevorrichtung 26 der Figuren 9 und 10 ist mit insgesamt drei ersten und drei zweiten Haltestrukturen 31, 32 versehen, um Sägeaufsätze unterschiedlicher Form und/oder Größe aufnehmen zu können. Die drei unterschiedlichen Haltestrukturen 31, 32 sind grundsätzlich ähnlich ausgebildet, so dass im Folgenden lediglich jeweils eine beschrieben wird.

Die erste Haltestruktur 31 besitzt einen Federarm 33, der eine Federbewegung in Richtung zur Grundplatte 30 ausführen kann. Der Federarm 33 ist auf seiner von der Grundplatte 30 abgewandten Seite mit einer Raststruktur 34 hier in Form einer Rastnase 34 versehen, die mit der Koppelstruktur 29 des Sägeaufsatzes 27 zusammenwirken kann und diesen derart relativ zur zweiten Haltestruktur 32 sowie in einer Richtung parallel zur Grundplatte 30 lagefixiert. Die zweite Haltestruktur 32 ist als Schlitz 35 ausgebildet, durch den die Koppelstruktur des Sägeaufsatzes 27 hindurchgeschoben werden kann, bis diese mit der ersten Haltestruktur 31 und der Nase 34 verrastet. Der Schlitz 35 ist derart dimensioniert, dass der Sägeaufsatz 27 quer zur Grundplatte 30 und in der verbleibenden Richtung parallel zu dieser lagepositioniert ist. Der Schlitz 35 und der Federarm 33 sind derart ausgebildet und zueinander positioniert, dass der Sägeaufsatz 27 nur unter Einfedern des Federarms 33 in seine bestimmungsgemäße Lage gebracht werden kann, in der er mit der Rastnase 34 verrastet ist.

Zum Entnehmen des Sägeaufsatzes 27 ist der Federarm 33 durch eine Bedienperson manuell in Richtung der Grundplatte 30 zu verformen, derart, dass die Koppelstruktur des Sägeaufsatzes 27 von der Rastnase 34 gelöst werden kann und dieser in einer Richtung parallel zur Grundplatte 30 aus dem Schlitz 35 herausgezogen werden kann. Ein gebrauchter Sägeaufsatz 27 kann besonders einfach zur Zwischenlagerung und/oder Entsorgung wieder in der Haltevorrichtung 26 angeordnet werden, indem er einfach wieder in Richtung der Rastnase 34 durch den Schlitz 35 geschoben wird, wobei der Federarm 33 einfedert, bis die Rastnase 34 mit der Koppelstruktur 29 des Sägeaufsatzes 27 verrastet und dieser in der Haltevorrichtung 26 lagegesichert ist.

Eine Aktivierung des Datenträgers 42 kann in der in den Figuren 9 und 10 gezeigten Ausführungsform wie folgt realisiert werden: Jede als eine Aufnahmebucht ausgebildete zweite Haltestruktur 32 kann als ein Taster ausgestaltet/ ausgebildet sein. Beispielsweise weist jede Aufnahmebucht aufgebrachte Leiterbahnen oder Litzen auf. Der Sägeaufsatz 27 ist eingerichtet/ ausgebildet, dass er einen Stromkreis schließen kann. Dies kann bevorzugt dadurch realisiert werden, dass der Sägeaufsatz 27 aus einem leitfähigen Material, insbesondere Metall, hergestellt ist oder zumindest leitfähige Schichten wie etwa ein aufgebrachtes Metall (in dem Fall, in welchem der Sägeaufsatz 27 etwa aus Keramik, Kunststoff oder Verbundwerkstoff hergestellt ist) aufweist. Beispielsweise kann bei einer Entnahme des Sägeaufsatzes 27 von der Haltvorrichtung 26 der Stromkreis durch den Sägeaufsatz 27 (kurzzeitig) geschlossen und der Datenträger 42 aktiviert werden.

### Bezugszeichenliste

- 1: Verpackung
- 2: Verpackung / Umverpackung
- 3: Unterschale
- 4: Vertiefung, Aufnahmeraum
- 5: Rand
- 6: Foliendeckel
- 7: Haltevorrichtung
- 8: Produkt, Werkzeug, Bohraufsatz
- 9: Wirkabschnitt, Bohrkopf
- 10: Koppelstruktur
- 11: Werkzeugschaft
- 12: Grundplatte
- 13: erste Haltestruktur
- 14: Haltearm
- 15: Haltearm
- 16: Schlitz
- 17: zweite Haltestruktur
- 18: Aufnahmeschlitz
- 19: Haltearm, Klemmarm
- 20: Haltearm, Klemmarm
- 21: Werkzeug, Fräsaufsatz
- 22: Wirkabschnitt, Fräskopf
- 23: dritte Haltestruktur
- 24: Einbuchtung
- 25: Einbuchtung
- 26: Haltevorrichtung
- 27: Werkzeug, Sägeaufsatz
- 28: Wirkabschnitt, Sägeblatt
- 29: Koppelstruktur
- 30: Grundplatte
- 31: erste Haltestruktur
- 32: zweite Haltestruktur
- 33: Federarm
- 34: Raststruktur, Rastnase
- 35: Schlitz
- 36: Schraubaufsatz
- 37: Schleifaufsatz
- 38: Schutzlasche
- 39: Schrägabschnitt
- 40: Endabschnitt
- 41: Standfläche
- 42: berührungslos auslesbarer / beschreibbarer Datenträger, RFID-Tag, NFC-Tag
- 43: Antennenkreis
- 44a, 44b: Kontakt

## Patentansprüche

1. Verpackung (1), insbesondere Sterilverpackung (1), für ein medizintechnisches Werkzeug (8, 21, 27), mit:
einer als Blisterverpackung ausgebildeten Umverpackung (2), welche eine Unterschale (3) mit einer einen Aufnahmeraum (4) für das medizintechnische Werkzeug (8, 21, 27) ausbildenden Vertiefung und eine an der Unterschale (3) angeordnete Deckelfolie (6) zum Verschließen des Aufnahmeraums (4) aufweist;
einer in dem Aufnahmeraum (4) angeordneten Haltevorrichtung (7, 26), in der das Werkzeug (8, 21, 27) gehalten ist; und
einem auslesbaren Datenträger (42) mit werkzeugspezifischen Daten, der an der Haltevorrichtung (7, 26) angeordnet ist.

2. Verpackung (1) nach Anspruch 1, **dadurch gekennzeichnet, dass** die Haltevorrichtung (7, 26) lagebestimmt in dem Aufnahmeraum (4) angeordnet ist und/oder das Werkzeug (8, 21, 27) lagebestimmt in der Haltevorrichtung (7, 26) gehalten ist und/oder der Datenträger (42) an der Haltevorrichtung (7, 26) aufgeklebt ist.

3. Verpackung nach Anspruch 1 oder 2, **dadurch gekennzeichnet, dass** der Datenträger (42) ein RFID-Tag (42) oder ein NFC-Tag (42) ist.

4. Verpackung (1) nach Anspruch 2 oder 3, **dadurch gekennzeichnet, dass** der Datenträger (42) und die Haltevorrichtung (7, 26) miteinander verklebt, vergossen oder verpresst sind.

5. Verpackung (1) nach einem der vorstehenden Ansprüche, **dadurch gekennzeichnet, dass** der Datenträger (42) durch eine Relativpositionierung des Werkzeugs (8, 21, 27) in der Haltevorrichtung (7, 26) aktivierbar ist, insbesondere durch eine derartige Relativpositionierung ein mit dem Datenträger (42) verbundener Antennenkreis (43) aktiviert wird.

6. Verpackung (1) nach einem der vorstehenden Ansprüche, **dadurch gekennzeichnet, dass** diese einen mit dem Datenträger (42) verbundenen Stromkreis und/oder Antennenkreis (43) aufweist.

7. Verpackung (1) nach einem der vorstehenden Ansprüche 1- 4, ferner mit einem mit dem Datenträger (42) verbundenen Antennenkreis (43) und/oder Stromkreis und/oder Schaltkreis, wobei der Antennenkreis (43) und/oder Stromkreis und/oder Schaltkreis durch eine Relativpositionierung des Werkzeugs (8, 21, 27) relativ zu der Haltevorrichtung (7, 26) aktivierbar ist, derart, dass bei in bestimmungsgemäßer Weise in der Haltevorrichtung (7, 26) positioniertem Werkzeug (8, 21, 27) der Antennenkreis (43) und/oder Stromkreis und/oder Schaltkreis offen, also inaktiv ist, und dass der Antennenkreis (43) und/oder Stromkreis und/oder Schaltkreis bei aus der bestimmungsgemäßen Lage entferntem Werkzeug geschlossen, also aktiviert ist.

8. Verfahren zum automatischen Identifizieren eines medizintechnischen Werkzeugs (8, 21, 27) mittels einer Verpackung (1) nach einem der vorstehenden Ansprüche, umfassend die folgenden Schritte:
- anbieterseitiges Einbringen des Werkzeugs (8, 21, 27) in die Haltevorrichtung (7, 26) und Einbringen der Haltevorrichtung (7, 26) in den Aufnahmeraum (4) der Verpackung (1),
- anbieterseitiges Beschreiben des Datenträgers (42) mit werkzeugspezifischen Daten,
- anbieterseitiges Verschließen der Verpackung (1),
- nutzerseitiges Öffnen der Verpackung (1),
- nutzerseitige Identifizierung des Werkzeugs (8, 21, 27) durch Auslesen des Datenträgers (42).

9. Verfahren nach Anspruch 8, **dadurch gekennzeichnet, dass** das Werkzeug (8, 21, 27) in der Haltevorrichtung (7, 26) angeordnet wird und der Datenträger (42) vor oder nach Anordnen des Werkzeugs (8, 21, 27) in der Haltevorrichtung (7, 26) vorzugsweise unter Sterilbedingungen beschrieben wird.

10. Verfahren nach Anspruch 8 oder 9, **dadurch gekennzeichnet, dass** nach dem Verschließen der Verpackung (1) eine γ-Sterilisation durchgeführt wird.

11. Verfahren nach einem der Ansprüche 8 bis 10, **dadurch gekennzeichnet, dass** der Datenträger beim Einbringen des in der Haltevorrichtung (7, 26) befindlichen Werkzeugs (8, 21, 27) in ein zur Aufnahme des Werkzeugs (8, 21, 27) bestimmtes Handstück durch eine vorzugsweise in dem Handstück befindliche Leseeinrichtung ausgelesen wird.

12. Verfahren nach einem der Ansprüche 8 bis 11, **dadurch gekennzeichnet, dass** aus dem Datenträger (42) ausgelesene Daten einem Anwender des Werkzeugs (8, 21, 27) dargestellt werden und/oder dass aus dem Datenträger (42) ausgelesene Daten des Werkzeugs (8, 21, 27) gespeichert und/oder an den Anbieter des Werkzeugs (8, 21, 27) geleitet werden, insbesondere automatisch geleitet werden, insbesondere automatisch in einer Datenbank gespeichert werden.

13. Verfahren nach einem der Ansprüche 8 bis 12, **dadurch gekennzeichnet, dass** der Datenträger (42) durch eine Relativpositionierung des Werkzeugs (8, 21, 27) in der Haltevorrichtung (7, 26) oder durch eine Entnahme daraus für ein Auslesen der auf dem Datenträger (42) befindlichen Daten oder ein Senden der auf dem Datenträger (42) befindlichen Daten aktiviert wird.

## Claims

1. A package (1), in particular a sterile package (1), for a medical tool (8, 21, 27) comprising:
an outer packaging (2) formed as a blister packaging having a lower shell (3) with a recess forming a receiving space (4) for the medical tool (8, 21, 27) and a lid foil (6) arranged on the lower shell for closing the receiving space (4);
a holding device (7, 26) arranged in the receiving space (4) in which the tool (8, 21, 27) is held; and
a readable data carrier (42) with tool-specific data, the data carrier being arranged on the holding device (7, 26).

2. The package (1) according to claim 1, **characterized in that** the holding device (7, 26) is arranged in the receiving space (4) in a position-determined manner, and/or the tool (8, 21, 27) is held in a position-determined manner in the holding device (7, 26), and/or the data carrier (42) is glued onto the holding device (7, 26).

3. The package (1) according to claim 1 or 2, **characterized in that** the data carrier (42) is an RFID tag (42) or an NFC tag (42).

4. The package (1) according to claim 2 or 3, **characterized in that** the data carrier (42) and the holding device (7, 26) are glued, cast or pressed together.

5. The package (1) according to any of the preceding claims, **characterized in that** the data carrier (42) is configured to be activated by relative positioning of the tool (8, 21, 27) in the holding device (7, 26), in particular an aerial circuit (43) connected with the data carrier (42) is activated by such relative positioning.

6. The package (1) according to any of the preceding claims, **characterized in that** the package comprises an electric circuit and/or antenna circuit (43) connected to the data carrier (42).

7. The package (1) according to any of claims 1-4, further comprising
an antenna circuit (43) and/or electric circuit and/or switching circuit connected to the data carrier (42), wherein the antenna circuit (43) and/or electric circuit and/or switching circuit can be activated by relative positioning of the tool (8, 21, 27) relative to the holding device (7, 26) in such a way that, when the tool (8, 21, 27) is positioned in the holding device (7, 26) in the manner intended, the antenna circuit (43) and/or electric circuit and/or switching circuit is open, i.e., inactive, and the antenna circuit (43) and/or electric circuit and/or switching circuit is closed, i.e., activated, when the tool is removed from its intended position.

8. A method for automatically identifying a medical tool (8, 21, 27) by means of a package (1) according to any of the preceding claims, the method comprising the following steps:
- inserting, on the provider side, the tool (8, 21, 27) in the holding device (7, 26) and inserting the holding device (7, 26) in the receiving space (4) of the package (1);
- writing, on the provider side, tool-specific data on the data carrier (42);
- closing the package (1) on the provider side;
- opening the package (1) on the user side; and
- identifying the tool (8, 21, 27) on the user side by reading out the data carrier (42).

9. The method according to claim 8, **characterized in that** the tool (8, 21, 27) is arranged in the holding device (7, 26) and data is written on the data carrier (42) before or after arranging the tool (8, 21, 27) in the holding device (7, 26), preferably under sterile conditions.

10. The method according to claim 8 or 9, **characterized in that** a γ sterilization is carried out after closing the package (1).

11. The method according to any of claims 8 to 10, **characterized in that** the data carrier is read out when the tool (8, 21, 27) located in the holding device (7, 26) is inserted in a handpiece for receiving the tool (8, 21, 27) by means of a reading device preferably located in the handpiece.

12. The method according to any of claims 8 to 11, **characterized in that** data read out from the data carrier (42) is displayed to a user of the tool (8, 21, 27) and/or that data of the tool (8, 21, 27) read out from the data carrier (42) is stored, in particular automatically in a database, and/or forwarded, in particular forwarded automatically, to the provider of the tool (8, 21, 27).

13. The method according to any of claims 8 to 12, **characterized in that** the data carrier (42) is activated by relative positioning of the tool (8, 21, 27) in the holding device (7, 26) or by removal therefrom for reading out the data located on the data carrier (42) or sending the data located on the data carrier (42).

## Revendications

1. Emballage (1), en particulier emballage stérile (1) pour un outil médical (8, 21, 27) avec :
un suremballage (2) formé comme emballage blister qui présente une coque inférieure (3) avec une cavité formant un espace de réception (4) pour l'outil médical (8, 21, 27) et un film de couvercle (6) agencé au niveau de la coque inférieure (3) pour la fermeture de l'espace de réception (4) ;
un dispositif de retenue (7, 26) agencé dans l'espace de réception (4), dispositif dans lequel l'outil (8, 21, 27) est maintenu ; et
un support de données (42) lisible avec des données spécifiques à l'outil, support qui est agencé au niveau du dispositif de retenue (7, 26).

2. Emballage (1) selon la revendication 1, **caractérisé en ce que** le dispositif de retenue (7, 26) est agencé dans une position déterminée dans l'espace de réception (4) et/ou l'outil (8, 21, 27) est maintenu dans une position déterminée dans le dispositif de retenue (7, 26) et/ou le support de données (42) est collé au dispositif de retenue (7, 26).

3. Emballage selon la revendication 1 ou 2, **caractérisé en ce que** le support de données (42) est une étiquette RFID (42) ou une étiquette NFC (42).

4. Emballage (1) selon la revendication 2 ou 3, **caractérisé en ce que** le support de données (42) et le dispositif de retenue (7, 26) sont collés, coulés ou pressés l'un avec l'autre.

5. Emballage (1) selon l'une quelconque des revendications précédentes, **caractérisé en ce que** le support de données (42) peut être activé par un positionnement relatif de l'outil (8, 21, 27) dans le dispositif de retenue (7, 26), en particulier un circuit d'antenne (43) relié au support de données (42) est activé par un tel positionnement relatif.

6. Emballage (1) selon l'une quelconque des revendications précédentes, **caractérisé en ce que** celui-ci présente un circuit électrique et/ou circuit d'antenne (43) relié au support de données (42).

7. Emballage (1) selon l'une quelconque des revendications précédentes 1 à 4, de plus avec un circuit d'antenne (43) et/ou circuit électrique et/ou circuit de commutation relié au support de données (42), dans lequel le circuit d'antenne (43) et/ou circuit électrique et/ou circuit de commutation peut être activé par un positionnement relatif de l'outil (8, 21, 27) par rapport au dispositif de retenue (7, 26) de telle manière que pour un outil (8, 21, 27) positionné de manière conforme à son usage prévu dans le dispositif de retenue (7, 26), le circuit d'antenne (43) et/ou circuit électrique et/ou circuit de commutation est ouvert, donc inactif, et en ce que le circuit d'antenne (43) et/ou circuit électrique et/ou circuit de commutation est fermé, donc activé, pour un outil éloigné de la position conforme à son usage prévu.

8. Procédé d'identification automatique d'un outil médical (8, 21, 27) au moyen d'un emballage (1) selon l'une quelconque des revendications précédentes, comprenant les étapes suivantes :
- l'introduction côté fournisseur de l'outil (8, 21, 27) dans le dispositif de retenue (7, 26) et l'introduction du dispositif de retenue (7, 26) dans l'espace de réception (4) de l'emballage (1),
- la description côté fournisseur du support de données (42) avec des données spécifiques à l'outil,
- la fermeture côté fournisseur de l'emballage (1),
- l'ouverture côté utilisateur de l'emballage (1),
- l'identification côté utilisateur de l'outil (8, 21, 27) par lecture du support de données (42).

9. Procédé selon la revendication 8, **caractérisé en ce que** l'outil (8, 21, 27) est agencé dans le dispositif de retenue (7, 26) et le support de données (42) est décrit avant ou après l'agencement de l'outil (8, 21, 27) dans le dispositif de retenue (7, 26) de préférence dans des conditions stériles.

10. Procédé selon la revendication 8 ou 9, **caractérisé en ce qu'**une stérilisation y est réalisée après la fermeture de l'emballage (1).

11. Procédé selon l'une quelconque des revendications 8 à 10, **caractérisé en ce que** le support de données est lu lors de l'introduction de l'outil (8, 21, 27) se trouvant dans le dispositif de retenue (7, 26) dans une pièce à main destinée à la réception de l'outil (8, 21, 27) par un dispositif de lecture se trouvant de préférence dans la pièce à main.

12. Procédé selon l'une quelconque des revendications 8 à 11, **caractérisé en ce que** des données lues à partir du support de données (42) sont représentées pour un utilisateur de l'outil (8, 21, 27) et/ou **en ce que** des données lues à partir du support de données (42) de l'outil (8, 21, 27) sont enregistrées et/ou sont acheminées vers le fournisseur de l'outil (8, 21, 27), en particulier sont acheminées automatiquement, en particulier sont enregistrées automatiquement dans une base de données.

13. Procédé selon l'une quelconque des revendications 8 à 12, **caractérisé en ce que** le support de données (42) est activé par un positionnement relatif de l'outil (8, 21, 27) dans le dispositif de retenue (7, 26) ou par un retrait de celui-ci pour une lecture des données se trouvant sur le support de données (42) ou un envoi des données se trouvant sur le support de données (42).
